# EUROPEAN PATENT APPLICATION

(11) **EP 4 278 961 A1**
(43) Date of publication of application: **22.11.2023**
(21) Application number: 22199754.7
(22) Date of filing: 05.10.2022
(51) Int. Cl.: A61B 5/021, A61B 5/022

(54) **PRESSURE SENSING ASSEMBLIES AND METHODS OF MAKING THE SAME**

(30) Priority: 19.05.2022 US 202263343611 P
(71) Applicant: Koninklijke Philips N.V., 5656 AG Eindhoven (NL)
(72) Inventor: PRUDDEN, JR, John, 5656AG Eindhoven (NL)
(74) Representative: Philips Intellectual Property & Standards

(57) **Abstract**

The present disclosure relates to pressure sensing assemblies (100) for use in non-invasive blood pressure devices and to methods of manufacturing such pressure sensing assemblies. As described herein, the pressure sensing assemblies (100) include an impermeable film (130) covering one or more cavities (108, 110) and/or channels (112) that are formed within a semi-rigid sheet (102). An interfacial connector (114) may sit within at least one of the cavities (108, 110) and/or channels (112) and connect the one or more cavities and/or channels to a tube assembly (118) located outside of the pressure sensing assembly (100). A fluid flow path, including the cavities (108, 110) and/or channel (112), defined by the pressure sensing assembly (100) is filled with at least a first pressure sensing fluid and used to hydraulically translate pressure changes generated by an adjacent blood vessel to an associated pressure transducing assembly of the non-invasive blood pressure device.

## Description

### FIELD OF THE DISCLOSURE

The present disclosure is directed generally to pressure sensing assemblies for non-invasive blood pressure cuffs, and more specifically, to methods of manufacturing such pressure sensing assemblies.

### BACKGROUND

In order to detect and record a patient's arterial blood pressure, a pressure sensing mechanism that is applied either invasively or non-invasively to the patient is needed. In one method of manufacturing non-invasive hard-shell blood pressure cuffs, a hard shell is formed into a cone shape and a sensor pad assembly is positioned inside the hard shell by rolling the sensor pad assembly and feeding it through a hole in the hard shell. Then, the sensor pad assembly can be filled with a pressure sensing fluid and glued into position onto the inside surface of the hard shell. However, these methods of manufacturing are complicated and introduce multiple points of potential failure for the end-device that may not be readily apparent or easily repaired.

### SUMMARY OF THE DISCLOSURE

The invention is defined by the independent claims. The dependent claims define advantageous embodiments.

According to an aspect of the present disclosure, a pressure sensing assembly is provided. The pressuring sensing assembly comprises: a semi-rigid sheet having a first cavity, a second cavity, and a channel connecting the first cavity and second cavity formed on a first surface of the semi-rigid sheet; an interfacial connector disposed within an aperture of the second cavity of the semi-rigid sheet; a tube assembly connected to the interfacial connector at a second surface of the semi-rigid sheet; and an impermeable film attached to at least a portion of the first surface of the semi-rigid sheet, wherein the impermeable film forms a fluid-tight seal over at least the first cavity, the second cavity, and the channel of the semi-rigid sheet.

Advantages of certain embodiments of the disclosed invention include that they provide a pressure sensing assembly having a reduced number of components compared to a known pressure sensing assembly, the cost is also reduced due to having fewer components, and they are more robust as a result of having fewer components which may potentially fail. Embodiments also reduce the errors that can occur with current manufacturing methods. Furthermore, embodiments resolve the challenge resulting from forming a hole in the semi rigid shell, which creates an unsupportive nature in the shell, and thus it needs to be filled.

In an embodiment, the tube assembly may comprise a tube and a first adapter, the interfacial connector being connected at a first end of the tube and the first adapter being connected to a second end of the tube.

In an embodiment, the pressure sensing assembly may form a continuous leak-free fluid path between the first cavity, the channel, the second cavity, and the tube assembly.

In an embodiment, the pressure sensing assembly may further comprise a first fluid contained within the continuous leak-free fluid path.

In an embodiment, the first fluid is a silicone oil.

In an embodiment, the pressure sensing assembly may further comprise at least one of a plurality of projections and/or a spacing device disposed within the first cavity of the semi-rigid sheet.

According to another aspect of the present disclosure, a non-invasive blood pressure cuff comprising the above pressure sensing assembly is provided.

In an embodiment, the non-invasive blood pressure cuff may further comprise a pressure transducing assembly having a second adapter connected to a pressure transducer, wherein the first adapter of the tube assembly is connected to the second adapter of the pressure transducing assembly.

According to still another aspect of the present disclosure, a method of manufacturing a pressure sensing assembly for a non-invasive blood pressure cuff is provided. The method comprises: (1) forming, on a first surface of a semi-rigid sheet, a first cavity, a second cavity, and a channel connecting the first cavity and the second cavity, wherein the second cavity comprises an aperture extending through the semi-rigid sheet to a second surface of the semi-rigid sheet; (2) securing an interfacial connector within the aperture of the second cavity; (3) connecting a tube assembly to the interfacial connector, wherein the tube assembly is adjacent to the second surface of the semi-rigid sheet; (4) attaching an impermeable film to at least a portion of the first surface of the semi-rigid sheet, wherein the impermeable film forms a fluid-tight seal over at least the first cavity, the second cavity, and the channel of the semi-rigid sheet; and (5) filling the first cavity, the second cavity, and the channel of the semi-rigid sheet and the tube of the tube assembly with a first fluid to form the pressure sensing assembly.

In an embodiment, the method may further comprise installing a spacing device within at least the first cavity of the semi-rigid sheet prior to attaching the impermeable film to the first surface of the semi-rigid sheet.

In an embodiment, the method may further comprise forming a plurality of projections within at least the first cavity of the semi-rigid sheet prior to attaching the impermeable film to the first surface of the semi-rigid sheet.

In an embodiment, one or more of the first cavity, the second cavity, and the channel may be formed using injection molding and/or thermoforming.

In an embodiment, connecting the tube assembly to the interfacial connector may form a fluid path between the tube assembly, the second cavity, the channel, and the first cavity.

In an embodiment, the tube assembly may comprise a tube and a first adapter, the interfacial connector being connected at a first end of the tube and the first adapter being connected to a second end of the tube. The method may then further comprise connecting the first adapter of the tube assembly to a second adapter of a pressure transducing assembly, wherein the pressure transducing assembly comprises the second adapter and a pressure transducer filled with a second fluid, the first and second adapters forming a leak-free boundary between the first fluid of the tube assembly and the second fluid of the pressure transducing assembly.

In an embodiment, the pressure transducing assembly may be disposable, and the first adapter of the tube assembly may be connected to a second adapter of the pressure transducing assembly.

These and other aspects and embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a partially exploded perspective view of a pressure sensing assembly illustrated according to aspects of the present disclosure.
FIG. 2 is a side view of a cross-section taken along the pressure sensing assembly illustrated according to aspects of the present disclosure.
FIG. 3 is a depiction of a tube assembly of a pressure sensing assembly illustrated according to aspects of the present disclosure.
FIG. 4A is a partially exploded view of a pressure sensing assembly illustrated according to aspects of the present disclosure.
FIG. 4B is a top plan view of an impermeable film used in a pressure sensing assembly and illustrated according to aspects of the present disclosure.
FIG. 5 is a perspective diagram of a portion of a pressure sensing assembly having a plurality of projections illustrated according to aspects of the present disclosure.
FIG. 6 is a top view of a diagram showing a spacing device within a cavity of a pressure sensing assembly illustrated according to aspects of the present disclosure.
FIG. 7 is a diagram of a non-invasive blood pressure cuff having a pressure sensing assembly and a pressure transducing assembly illustrated according to aspects of the present disclosure.
FIG. 8 is a flowchart illustrating a method of manufacturing a pressure sensing assembly for use in a non-invasive blood pressure cuff according to aspects of the present disclosure.

### DETAILED DESCRIPTION OF EMBODIMENTS

The present disclosure relates to non-invasive blood pressure cuffs, pressure sensing assemblies for use in non-invasive blood pressure cuffs, and methods of manufacturing such devices. When a blood pressure cuff of the present disclosure is non-invasively applied to a patient, a pressure sensing assembly of the blood pressure cuff hydraulically translates arterial pressure changes generated by an arterial pulse. Further, as described herein, the pressure sensing assembly can be reversibly connected to a pressure transducer to measure and record the patient's blood pressure.

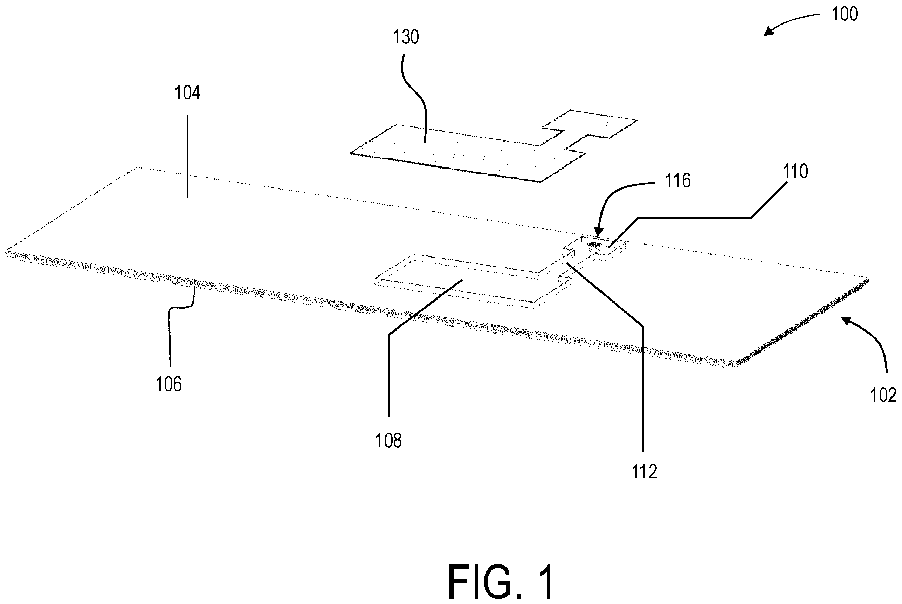

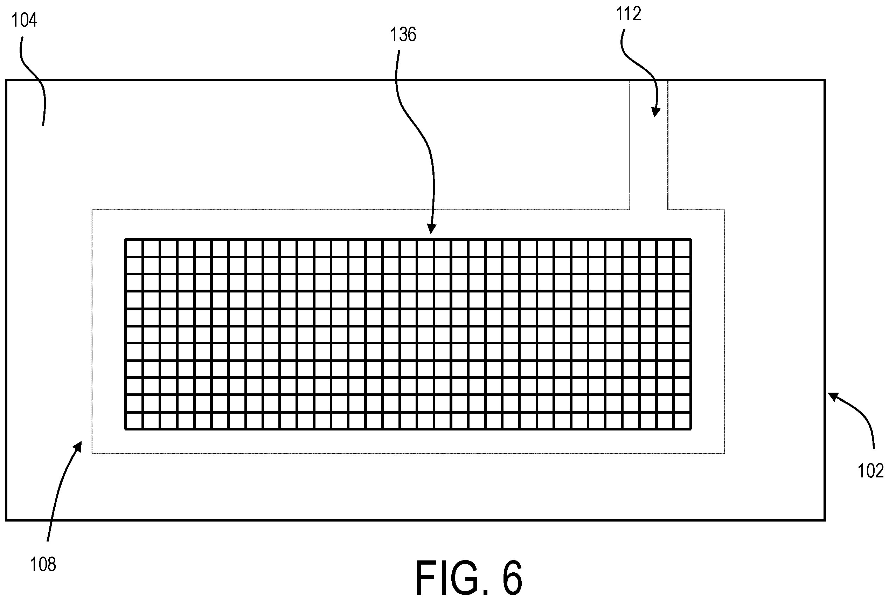

Turning to FIG. 1, a pressure sensing assembly 100 is provided according to various aspects of the present disclosure. As shown, the pressure sensing assembly 100 can include a sheet 102 having at least a first surface 104 and a second surface 106. The sheet 102 is a semi-rigid sheet comprising a polymer composition that, when heated, may be bent, rolled, and/or curled into a circular configuration (as shown in FIG. 6), but otherwise maintains a high stiffness. The sheet 102 can have a thickness of from about 0.5 millimeters to about 5 mm, including about 0.5 mm, about 1 mm, about 1.5 mm, about 2 mm, about 2.5 mm, about 3 mm, about 3.5 mm, about 4 mm, about 4.5 mm, about 5 mm, and ranges formed from any combination of endpoints thereof. Although illustrated as having a rectangular shape, the sheet 102 can have other shapes and is not limited to any particular geometry except as otherwise discussed herein.

In embodiments, the first surface 104 of the sheet 102 can be placed against a patient's body (e.g., around a patient's upper arm) when used part of the pressure sensing assembly 100 of a non-invasive blood pressure cuff 200. As such, when used as part of a pressure sensing assembly 100 of a non-invasive blood pressure cuff 200, the sheet 102 provides a rigid structure against which an arterial pulse can be hydraulically translated to the pressure sensing assembly 100.

According to aspects of the present disclosure, the first surface 104 of the sheet 102 has one or more cavities and channels formed thereon. For example, the first surface 104 may have at least a first cavity 108, a second cavity 110, and a channel 112 connecting the first and second cavities 108, 110. In embodiments, each of the cavities 108, 110 and/or channel(s) 112 can be formed along with the sheet 102 using injection molding and/or thermoforming. In further embodiments, the cavities 108, 110 and channel 112 can have a depth less than the total thickness of the sheet 102. In other embodiments, the cavities 108, 110 and channel 112 may form projections extending from the second surface of the sheet 102. Although illustrated as having rectangular shapes, the cavities 108, 110 and channel 112 can have other shapes and is not limited to any particular geometry except as otherwise discussed herein. For example, and without limitation, the cavities 108, 110 can be circular and/or have rounded or curved edges to facilitate pressure sensing features when the sheet 102 is rolled into a circular configuration.

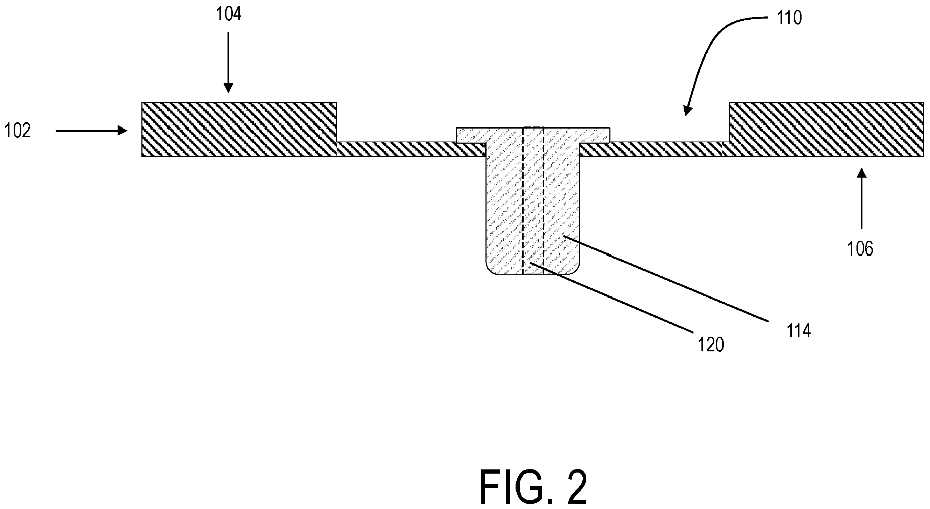

In embodiments, at least one of the cavities 108, 110 include an aperture 116 extending through the entire thickness of the sheet 102 (i.e., through to the second surface 106 from the first surface 104). In specific embodiments, the second cavity 110 may include the aperture 116, as shown in FIG. 1 and FIG. 2. Further, as shown in FIG. 2, the pressure sensing assembly 100 includes an interfacial connector 114 that is placed or secured within the aperture 116. The interfacial connector 114 can be, for example and without limitation, a tube fitting or other adapter having a bore 120 configured to receive and/or secure at least a portion of a tube.

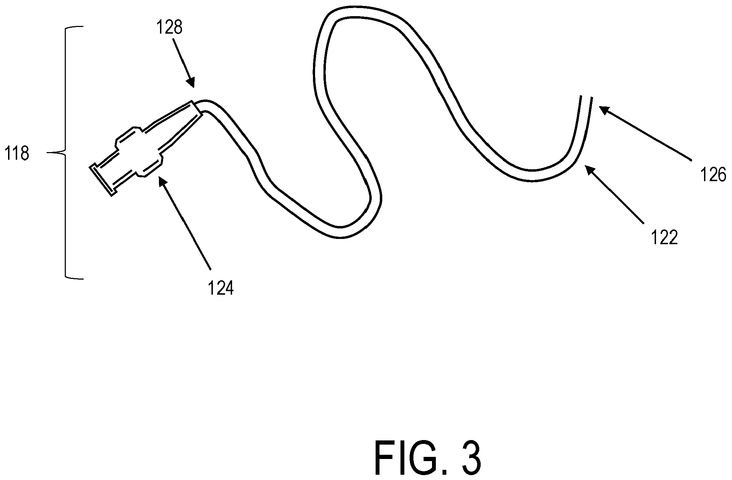

For example, with reference to FIG. 3, the pressure sensing assembly 100 includes a tube assembly 118 that is connected to the interfacial connector 114 at the second surface 106 of the sheet 102. The tube assembly 118 can include at least a tube 122 and a first adapter 124. In certain aspects, the interfacial connector 114 can be connected to a first end 126 of the tube 122 while a second end 128 of the tube 122 is connected to the first adapter 124. In embodiments, the first adapter 124 is a fluid-tight adapter, such as a luer lock adapter or a portion thereof. In specific embodiments, the first adapter 124 can be a female luer lock adapter.

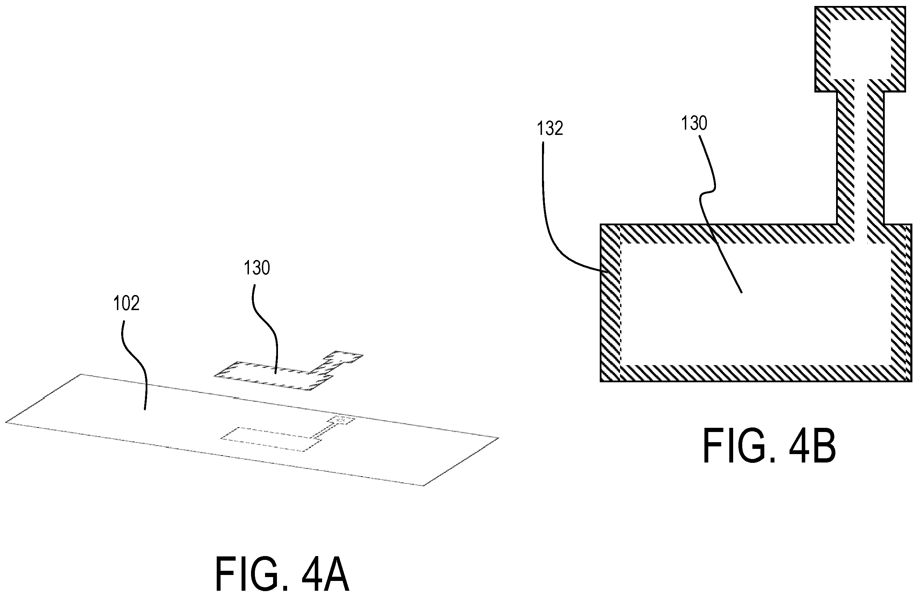

Turning to FIGS. 4A and 4B, the pressure sensing assembly 100 further includes a film (e.g. a membrane) 130 attached to at least a portion of the first surface 104 of the sheet 102. As shown in FIG. 4A, the pressure sensing assembly 100 is illustrated in a partially exploded view with the film 130 above the cavities 108, 110 and channel 112. In specific embodiments, the film 130 can cover at least the cavities 108, 110 and channel 112. For example, the film 130 may cover the cavities 108, 110 and channel 112 and a certain amount 132 of the first surface 104 around the cavities 108, 110 and channel 112.

The film 130 may be attached to the first surface 104 of the sheet 102 in a variety of ways. For example, as shown in FIG. 4B, the film 130 may be attached to the first surface 104 of the sheet 102 via an adhesive running along the perimeter 132 of the film 130. Although the film 130 is illustrated with a geometry that approximates the shapes of the cavities 108, 110 and channel 112, the film 130 can have other shapes and sizes unless otherwise discussed herein.

In particular, however, the film 130 forms a fluid-tight seal over one or more of the cavities 108, 110 and channel 112. With the film 130 attached to the surface 104 of the sheet 102, the pressure sensing assembly 100 forms a continuous, leak-free fluid path between the one or more cavities and/or channels, such as the first cavity 108, the channel 112, and the second cavity 110. Thus, the film 130 is an impermeable membrane, with the notion "impermeable" meaning that the film prevents at least a first fluid from escaping the fluid path. In particular embodiments, this fluid path can further include the tube assembly 118 (e.g., the tube 122 and/or the first adapter 124).

In accordance with various aspects of the present disclosure, the fluid path enclosed by the sheet 102 and the film 130 can be filled with at least a first fluid, which can be a pressure sensing fluid. In other words, the pressure sensing assembly 100 can include at least a first pressure sensing fluid that fills one or more cavities and/or channels (e.g., the cavities 108, 110 and channel 112). As such, the film 130 may be impermeable to at least the first fluid, including at least the first pressure sensing fluid. In embodiments, at least the first pressure sensing fluid comprises a liquid or aqueous solution capable of translating a pressure differential generated by the flow of blood in an adjacent blood vessel along the fluid path. In specific embodiments, the first pressure sensing fluid can include a silicone oil.

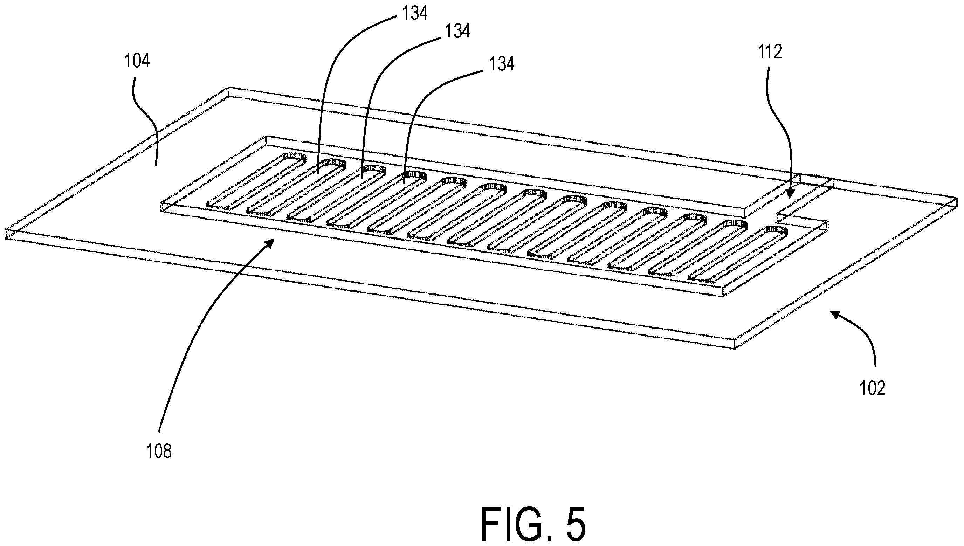

Turning now to FIGS. 5 and 6, at least a portion of the fluid path can include a spacing device and/or a plurality of projections. That is, a plurality of projections 134 and/or a spacing device 136 can be formed and/or disposed within at least a portion of the space defining the fluid path. For example, and without limitation, a plurality of projections 134 may be formed and/or disposed within one or more of the cavities and/or channels of the sheet 102 (e.g., the first cavity 108, the second cavity 110, and/or the channel 112). Alternatively, or in combination with the projections 134, a spacing device 136 can be formed and/or disposed within one or more of the cavities and/or channels of the sheet 102 (e.g., the first cavity 108, the second cavity 110, and/or the channel 112). In embodiments, the projections 134 and/or the spacing device 136 can be used to prevent the film 130 from sticking or adhering to the cavities 108, 110 and/or channel 112.

As shown in FIG. 5, a plurality of projections 134 are formed within the first cavity 108 that will allow the first fluid to flow through the fluid path as discussed above, but will also prevent the film 130 (not shown in FIG. 5) from adhering to the cavity 108. Similarly, in FIG. 6, a spacing device 136 is disposed within the first cavity 108 that allows the first fluid to flow through the fluid path as discussed above while also preventing the film 130 (not shown in FIG. 6) from adhering to the cavity 108. In particular embodiments, the spacing device 136 may be a mesh screen.

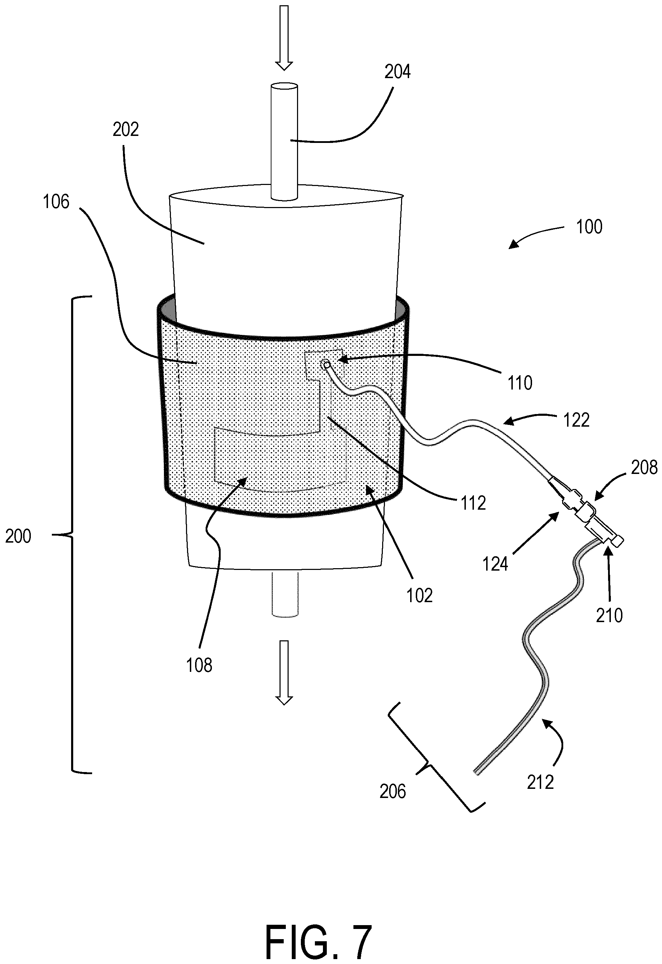

According to further aspects of the present disclosure, a non-invasive blood pressure cuff 200 comprising a pressure sensing assembly 100 is provided. With reference to FIG. 7, one such non-invasive blood pressure cuff 200 is illustrated as it may be applied to a patient's body part 202 (e.g., a patient's upper arm).

As discussed below, the pressure sensing assembly 100 may be heated and curled, rolled, or otherwise bent into a circular configuration during manufacturing (i.e., prior to applying the cuff 200 to the patient's body 202). In embodiments, the one or more cavities and channels (e.g., cavities 108, 110 and the channel 112) may be formed while the sheet 102 of the pressure sensing assembly 100 is curled or uncurled. Similarly, the film 130 may be attached and the fluid path may be filled while the sheet 102 of the pressure sensing assembly 100 is curled or uncurled. When curled, however, the sheet 102 and/or the non-invasive blood pressure cuff 200 can have a rounded and/or a substantially circular cross-section. Once the pressure sensing assembly 100 is curled, the sheet 102 is flexible enough to allow placement on the patient's body 202 but rigid enough that pressure differentials generated by the flow of blood through a blood vessel 204 adjacent to the pressure sensing assembly 100 can be hydraulically translated to the pressure sensing fluid of the pressure sensing assembly 100.

As shown in FIG. 7, the pressure sensing assembly 100 is curled such that the second surface 106 of the sheet 102 faces away from the patient's body part 202. In other words, the first surface 104 of the sheet 102 non-invasively contacts the surface of the patient's body part 202. When an arterial pulse is generated in a blood vessel 204 adjacent to the non-invasive blood pressure cuff 200, a resultant wave / signal can propagate through the pressure sensing fluid filling the fluid path.

As also shown in FIG. 7, the non-invasive blood pressure cuff 200 can further comprise a pressure transducing assembly 206. The pressure transducing assembly 206 can include, but is not limited to, a second adapter 208 configured to connect to the first adapter 124 of the tube assembly 118, a pressure transducer 210 configured to convert changes in pressure to an electrical signal, and/or a cable 212 configured to measure electrical signals using the pressure transducer 210. In some embodiments, the second adapter 208 may be a fluid-tight adapter, such as a luer lock adapter or a portion thereof. In specific embodiments, the second adapter 208 can be a male luer lock adapter configured to engage the first adapter 124 of the tube assembly 118.

In other embodiments, the tube assembly 118 may not include a first adapter 124, and the tube 122 of the tube assembly 118 is directly connected to the pressure transducing assembly 206. For example, the second end 128 of the tube 122 may be directly connected to the pressure transducer 210 of the pressure transducing assembly 206. In some embodiments, the tube assembly 118 may be irreversibly connected and/or secured to the pressure transducing assembly 206.

In embodiments, the pressure transducing assembly 206 may contain or otherwise comprise at least a second fluid. For example, the pressure transducer 210 of the pressure transducing assembly 206 can be filled with an amount of at least the second fluid. In some embodiments, the second fluid of the pressure transducing assembly 206 can be at least a second pressure sensing fluid. In embodiments, at least the second pressure sensing fluid comprises a liquid or aqueous solution capable of translating a pressure differential generated by the flow of at least the first fluid along the fluid path of the pressure sensing assembly 100. In specific embodiments, the second pressure sensing fluid can include glycerin.

In embodiments, the first and second adapters 124, 208 may form a leak-free boundary between at least the first fluid of the tube assembly 118 and at least the second fluid of the pressure transducing assembly 206. Thus, when the first and second adapters 124, 208 are connected, the different fluids (e.g., at least the first and second fluids) will not mix. In embodiments where the tube assembly 118 does not include a first adapter 124 and is irreversibly connected to the pressure transducing assembly 206, the irreversible connection may form the leak-free boundary between at least the first fluid of the tube assembly 118 and at least the second fluid of the pressure transducing assembly 206.

However, in certain embodiments, the first and second adapters 124, 208 may be reversibly connectable such that the pressure transducing assembly 206 can be disconnected from the pressure sensing assembly 100. In specific embodiments, the pressure transducing assembly 206 or one or more parts of the pressure transducing assembly 206 can be disposable. For example, in some embodiments, the cable 212, the pressure transducer 210 and/or the second adapter 208 may be disposable.

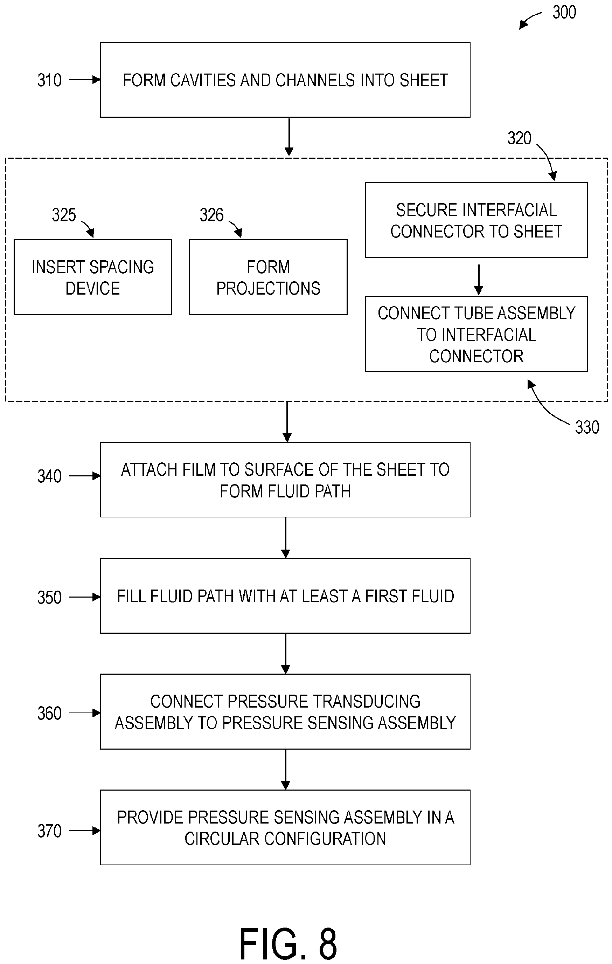

Turning now to FIG. 8, also provided herein are methods of manufacturing a pressure sensing assembly 100 for a non-invasive blood pressure cuff 200. According to an embodiment of the present disclosure, a method 300 of manufacturing a pressure sensing assembly 100 for a non-invasive blood pressure cuff 200 includes the following steps: forming 310, on a first surface 104 of a semi-rigid sheet 102, a first cavity 108, a second cavity 110, and a channel 112 connecting the first cavity 108 and the second cavity 110, wherein the second cavity 110 comprises an aperture 116 extending through the semi-rigid sheet 102 to a second surface 106 of the semi-rigid sheet 102; securing 320 an interfacial connector 114 within the aperture 116 of one of the cavities 108, 110; connecting 330 a tube assembly 118 to the interfacial connector 114, wherein the tube assembly 118 is adjacent to the second surface 106 of the semi-rigid sheet 102; attaching 340 an impermeable film 130 to at least a portion of the first surface 104 of the semi-rigid sheet 102, wherein the impermeable film 130 forms a fluid-tight seal over at least the first cavity 108, the second cavity 110, and the channel 112 of the semi-rigid sheet 102; filling 350 the first cavity 108, the second cavity 110, and the channel 112 of the semi-rigid sheet 102 and the tube 122 of the tube assembly 118 with at least a first fluid to form the pressure sensing assembly 100.

More particularly, in a first step, the method 300 includes forming 310 one or more cavities and/or channels (e.g., cavities 108, 110, channel 112, etc.) on a first surface 104 of a semi-rigid sheet 102. The one or more channels and cavities may be interconnected such that when fully assembled, these cavities and channels (e.g., cavities 108, 110, channel 112, etc.) define a fluid flow path for at least a first pressure sensing fluid. In embodiments, one or more of the cavities 108, 110 and/or channels 112 may include an aperture 116 extending through the sheet 102.

In embodiments, each of the one or more cavities and channels (e.g., cavities 108, 110, channel 112, etc.) may be formed 310 either using injection molding, thermoforming, three-dimensional printing, or another similar production technique. For example, in an injection molding process, a composition is heated to become a molten composition that is then injected through a nozzle under a certain pressure into a prefabricated mold. Once the molten composition is cured, the molded part can be removed from the mold for further processing. In a thermoforming process, a sheet formed from a composition is heated to a forming temperature and applied to a mold using pressure forming, vacuum forming, mechanical forming, or the like. The sheet material is then cooled down and trimmed to result in the finished part. Alternatively, in a three-dimensional printing process, a digital model is used to deposit, join, and/or solidify a material composition (e.g., in the form of a solid, liquid, powder, or the like) in a plurality of layers or operations.

In particular embodiments, the compositions used in the forming step 310 can comprise a polymer composition, including but not limited to, an acrylate, a polyethylene, a polystyrene, Lexan, a polycarbonate, a polypropylene, polyethylene terephthalate glycol, acrylonitrile butadiene styrene, polymethyl methacrylate, polyvinyl chloride, and the like. In certain embodiments, the compositions used in the forming step 310 can include a combination of two or more polymers, or can include one or more additives such as pigments, fillers, and/or strengtheners.

In embodiments, the cavities 108, 110 and/or channels 112 may be formed after the sheet 102 is formed or at the same time as the sheet 102. Further, the cavities 108, 110 and/or channels 112 may be formed while the sheet 102 is unrolled (i.e., flat) or while the sheet 102 is curled (i.e., rolled into a circular configuration).

In further embodiments, at least one of the cavities and/or channels (e.g., cavities 108, 110, channel 112, etc.) can comprise an aperture 116 extending through the semi-rigid sheet 102 to a second surface 106 of the semi-rigid sheet 102.

In a second step, the method 300 includes securing 320 at least one interfacial connector 114 within an aperture 116 of the pressure sensing assembly 100. In embodiments, the interfacial connector may be a tube fitting such as connector 114 shown in FIG. 2. The interfacial connector 114 may be secured 320 within the aperture 116 such that it forms a fluid-tight seal over the corresponding aperture 116. In embodiments, the interfacial connector 114 may include a hollow bore 120 that is configured to receive a tube 122. Although illustrated in FIG. 2 as a separate part, the interfacial connector 114 or a portion thereof may be formed along with the cavities 108, 110 and/or channels 112 during step 310. Alternatively, the connector 114 may be irreversibly secured within the corresponding aperture 116 through a bonding process.

Next, the method 300 includes connecting 330 a tube assembly (e.g., tube assembly 118) to the interfacial connector 114. In embodiments, the tube assembly 118 can include at least a first adapter 128 as well as a tube 122 having a first end 126 and a second end 128. The tube assembly 118 may be connected to the interfacial connector 114 such that the tube assembly 118 is adjacent to the second surface 106 of the sheet 102. In other words, when the pressure sensing assembly 100 is curled into a circular configuration and applied to a patient's body part 202, the first surface 104 faces the patient's body part 202, the second surface 106 faces away from the patient's body part 202, and the tube assembly 118 is outside of the second surface 106 of the sheet 102.

The method 300 then includes attaching 340 a film (e.g., film 130) to at least a portion of the first surface 104 of the sheet 102 such that the film 130 forms a fluid-tight seal over at least one the more cavities 108, 110 and/or channels 112. The film 130 may be attached to the sheet 102 via an adhesive, as discussed above, although other methods are contemplated.

After being attached, the film 130 forms a fluid path between the one or more cavities 108, 110 and channels 112 and the tube assembly 118. Thus, in a step 350, the method 300 includes filling the formed fluid path with at least a first fluid to form the pressure sensing assembly 100. In embodiments, the film 130 may be a flexible thin film capable of imparting pressure differentials generated by an adjacent blood vessel 204 to the fluid, which may be translated through the one or more cavities 108, 110 and channels 112 and through the tube assembly 118.

In particular embodiments, the method 300 can further include steps 325 and/or 326, wherein a spacing device 136 is placed and/or a plurality of projections 134 are formed with one or more of the cavities 108, 110 and/or channels 112 of the sheet 102. In particular embodiments, a spacing device 136 can be placed only within the first cavity 108 of the sheet 102. In other embodiments, a plurality of projections 134 are formed only within the first cavity 108. In still further embodiments, the cavities 108, 110 and/or channels 112 may include a combination of spacing devices 136 and/or projections 134. As shown in FIG. 8, steps 325, 326 can be performed individually and/or simultaneously with one or more other steps of the method 300, including but not limited to steps 320, 330. In certain embodiments, for example, the plurality of projections 134 may be formed 326 at the same time as the one or more cavities 108, 110 and channels 112 in step 310. In other embodiments, the steps 325, 326 are performed before attaching the film 130 in step 340.

Next, in a step 360, the method 300 can include connecting the pressure sensing assembly 100 to a pressure transducing assembly 206. In embodiments, the pressure transducing assembly 206 can comprise at least a second adapter 208 and a pressure transducer 210 comprising at least a second fluid as discussed above, such that the step 360 includes connecting the second adapter 208 to the first adapter 124. The second fluid may be a pressuring sensing fluid like the first fluid, may be the same fluid as the first fluid, or may be a different fluid than the first fluid. By connecting the pressure sensing assembly 100 to the pressure transducing assembly 206, the adapters 124, 208 can form a leak-free boundary between the first fluid of the tube assembly 118 and the second fluid of the pressure transducing assembly 206. In some embodiments, the pressure transducing assembly 206 or a component thereof may be disposable and the first adapter 124 of the tube assembly 118 may be reversibly connected to the second adapter 208 of the pressure transducing assembly 206. In specific embodiments, the first adapter 124 can be a female luer lock adapter and the second adapter 208 can be a male luer lock adapter.

Finally, in certain embodiments, the method 300 may include a step 370 wherein the pressure sensing assembly 100 is curled, rolled, or otherwise formed into a circular configuration that can be worn on a body part 202 of a patient as illustrated in FIG. 7. As discussed above, the pressure sensing assembly 100 may be formed while already in a circular configuration. In other embodiments, the sheet 102 of the pressure sensing assembly 100 may be formed (e.g., one or more of the steps 310-360) while the sheet 102 is flat, and then subsequently formed into a circular configuration in a step 370. In further embodiments, the sheet 102 may be formed into a circular configuration first, and then reversibly unrolled to perform one or more of the steps 310-360. As such, it is contemplated that one or more of the steps 310-360 are performed while the pressure sensing assembly 100 is in a circular or flat configuration.

As discussed herein, the pressure sensing assembly 100 may form part of a non-invasive blood pressure cuff 200 that can be placed around a patient's body part 202 such that a blood pressure of the patient can be measured. More specifically, the first surface 104 of the pressure sensing assembly 100 of the blood pressure cuff 200 may be placed around the patient's body part 202 adjacent to a blood vessel 204, such as an artery. When the arterial pressure changes, such as due to the flow of blood through the vessel 204, the pressure is hydraulically translated through the pressure sensing fluids of the pressure sensing assembly 100 and the pressure transducing assembly 206.

It should be appreciated that the pressure sensing assemblies of the present disclosure are not limited the particular shapes, dimensions, and/or geometries shown in the figures. As such, other combinations and configurations are expressly contemplated. It should also be appreciated that all combinations of the foregoing concepts and additional concepts discussed herein (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "having," "containing," and the like are to be understood to be open-ended, i.e., to mean including but not limited to.

It should also be understood that, unless clearly indicated to the contrary, in any methods claimed herein that include more than one step or act, the order of the steps or acts of the method is not necessarily limited to the order in which the steps or acts of the method are recited.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

In the claims, any reference signs placed between parentheses shall not be construed as limiting the claim. Measures recited in mutually different dependent claims may advantageously be used in combination.

## Claims

1. A pressure sensing assembly (100), the assembly (100) comprising:
a semi-rigid sheet (102) having a first cavity (108), a second cavity (110), and a channel (112) connecting the first cavity (108) and second cavity (110) formed on a first surface (104) of the semi-rigid sheet (102);
an interfacial connector (114) disposed within an aperture (116) of the second cavity of the semi-rigid sheet (102);
a tube assembly (118) connected to the interfacial connector (114) at a second surface (106) of the semi-rigid sheet (102); and
an impermeable film (130) attached to at least a portion of the first surface (104) of the semi-rigid sheet (102), wherein the impermeable film forms a fluid-tight seal over at least the first cavity (108), the second cavity (110), and the channel (112) of the semi-rigid sheet (102).

2. The pressure sensing assembly (100) of claim 1, wherein the tube assembly (118) comprises a tube (122) and a first adapter (124), the interfacial connector (114) being connected at a first end (126) of the tube (122) and the first adapter (124) being connected to a second end (128) of the tube (122).

3. The pressure sensing assembly (100) of claim 1, wherein the pressure sensing assembly (100) forms a continuous leak-free fluid path between the first cavity (108), the channel (112), the second cavity (110), and the tube assembly (118).

4. The pressure sensing assembly (100) of claim 3, further comprising a first fluid contained within the continuous leak-free fluid path.

5. The pressure sensing assembly (100) of claim 4, wherein the first fluid is a silicone oil.

6. The pressure sensing assembly (100) of claim 1, further comprising at least one of a plurality of projections (134) and/or a spacing device (136) disposed within the first cavity (108) of the semi-rigid sheet (102).

7. A non-invasive blood pressure cuff (200) comprising a pressure sensing assembly (100) according to any one of claims 1-6.

8. The non-invasive blood pressure cuff (200) of claim 7, further comprising a pressure transducing assembly (206) having a second adapter (208) connected to a pressure transducer (210), wherein the first adapter (124) of the tube assembly (118) is connected to the second adapter (208) of the pressure transducing assembly (206).

9. A method (300) of manufacturing a pressure sensing assembly (100) for a non-invasive blood pressure cuff (200), the method comprising:
forming (310), on a first surface (104) of a semi-rigid sheet (102), a first cavity (108), a second cavity (110), and a channel (112) connecting the first cavity (108) and the second cavity (110), wherein the second cavity (110) comprises an aperture (116) extending through the semi-rigid sheet (102) to a second surface (106) of the semi-rigid sheet (102);
securing (320) an interfacial connector (114) within the aperture (116) of the second cavity (110);
connecting (330) a tube assembly (118) to the interfacial connector (114), wherein the tube assembly (118) is adjacent to the second surface (106) of the semi-rigid sheet (102);
attaching (340) an impermeable film (130) to at least a portion of the first surface (104) of the semi-rigid sheet (102), wherein the impermeable film (130) forms a fluid-tight seal over at least the first cavity (108), the second cavity (110), and the channel (112) of the semi-rigid sheet (102);
filling (350) the first cavity (108), the second cavity (110), and the channel (112) of the semi-rigid sheet (112) and the tube assembly (118) with a first fluid to form the pressure sensing assembly (100).

10. The method (300) of claim 9, further comprising:
installing (325) a spacing device (136) within at least the first cavity (108) of the semi-rigid sheet (102) prior to attaching the impermeable film (130) to the first surface (104) of the semi-rigid sheet (102).

11. The method (300) of claim 9, further comprising:
forming (326) a plurality of projections (134) within at least the first cavity (108) of the semi-rigid sheet (102) prior to attaching the impermeable film (130) to the first surface (104) of the semi-rigid sheet (102).

12. The method (300) of claim 9, wherein one or more of the first cavity (108), the second cavity (110), and the channel (112) are formed using injection molding and/or thermoforming.

13. The method (300) of claim 9, wherein connecting the tube assembly (118) to the interfacial connector (114) forms a fluid path between the tube assembly (118), the second cavity (110), the channel (112), and the first cavity (108).

14. The method (300) of claim 9, wherein the tube assembly (118) comprises a tube (122) and a first adapter (124), the interfacial connector (114) being connected at a first end (126) of the tube (122) and the first adapter (124) being connected to a second end (128) of the tube (122), and wherein the method (300) further comprises:
connecting (360) the first adapter (124) of the tube assembly (118) to a second adapter (208) of a pressure transducing assembly (206), wherein the pressure transducing assembly (206) comprises the second adapter (208) and a pressure transducer (210) filled with a second fluid, the first and second adapters (124, 208) forming a leak-free boundary between the first fluid of the tube assembly (118) and the second fluid of the pressure transducing assembly (206).

15. The method (300) of claim 14, wherein the pressure transducing assembly (206) is disposable and the first adapter (124) of the tube assembly (118) is connected to the second adapter (208) of the pressure transducing assembly (206).
